# EUROPEAN PATENT APPLICATION

(11) **EP 1 897 554 A1**
(43) Date of publication of application: **12.03.2008**
(21) Application number: 06017750.8
(22) Date of filing: 25.08.2006
(51) Int. Cl.: A61K 38/49, A61P 9/10

(54) **Method for the manufacture of a medicament for treating hemorrhagic stroke**

(71) Applicant: PAION Deutschland GmbH, 52062 Aachen (DE)
(72) Inventor: Thiex, Ruth, 52074 Aachen (DE); Rohde, Veith, 37085 Göttingen (DE); Petersen, Karl-Uwe, 52072 Aachen (DE)
(74) Representative: Von Renesse, Dorothea

(57) **Abstract**

Use of a plasminogen activator for the manufacture of a medicament for the treatment of hemorrhagic stroke, wherein the plasminogen activator does not substantially activate the NMDA type glutamate receptor within the patient.

### Annex to the application documents - subsequently filed sequences listing

SEQUENCE LISTING

<110>  PAION Deutschland GmbH

<120>  Method for the manufacture of a medicament for treating
       hemorrhagic stroke

<130>  47 062 K

<140>  06 017 750.8
<141>  2006-08-25

<160>  1

<170>  PatentIn version 3.3

<210>  1
<211>  441
<212>  PRT
<213>  Desmodus rotundus

<400>  1

Ala Tyr Gly Val Ala Cys Lys Asp Glu Ile Thr Gln Met Thr Tyr Arg
1               5                   10                  15

Arg Gln Glu Ser Trp Leu Arg Pro Glu Val Arg Ser Lys Arg Val Glu
            20                  25                  30

His Cys Gln Cys Asp Arg Gly Gln Ala Arg Cys His Thr Val Pro Val
        35                  40                  45

Asn Ser Cys Ser Glu Pro Arg Cys Phe Asn Gly Gly Thr Cys Trp Gln
    50                  55                  60

Ala Val Tyr Phe Ser Asp Phe Val Cys Gln Cys Pro Ala Gly Tyr Thr
65                  70                  75                  80

Gly Lys Arg Cys Glu Val Asp Thr Arg Ala Thr Cys Tyr Glu Gly Gln
                85                  90                  95

Gly Val Thr Tyr Arg Gly Thr Trp Ser Thr Ala Glu Ser Arg Val Glu
            100                 105                 110

Cys Ile Asn Trp Asn Ser Ser Leu Leu Thr Arg Arg Thr Tyr Asn Gly
        115                 120                 125

Arg Met Pro Asp Ala Phe Asn Leu Gly Leu Gly Asn His Asn Tyr Cys
    130                 135                 140

Arg Asn Pro Asn Gly Ala Pro Lys Pro Trp Cys Tyr Val Ile Lys Ala
145                 150                 155                 160

Gly Lys Phe Thr Ser Glu Ser Cys Ser Val Pro Val Cys Ser Lys Ala
                165                 170                 175

Thr Cys Gly Leu Arg Lys Tyr Lys Glu Pro Gln Leu His Ser Thr Gly

            180                 185                 190

Gly Leu Phe Thr Asp Ile Thr Ser His Pro Trp Gln Ala Ala Ile Phe
        195                 200                 205

Ala Gln Asn Arg Arg Ser Ser Gly Glu Arg Phe Leu Cys Gly Gly Ile
    210                 215                 220

Leu Ile Ser Ser Cys Trp Val Leu Thr Ala Ala His Cys Phe Gln Glu
225                 230                 235                 240

Ser Tyr Leu Pro Asp Gln Leu Lys Val Val Leu Gly Arg Thr Tyr Arg
                245                 250                 255

Val Lys Pro Gly Glu Glu Glu Gln Thr Phe Lys Val Lys Lys Tyr Ile
            260                 265                 270

Val His Lys Glu Phe Asp Asp Asp Thr Tyr Asn Asn Asp Ile Ala Leu
        275                 280                 285

Leu Gln Leu Lys Ser Asp Ser Pro Gln Cys Ala Gln Glu Ser Asp Ser
    290                 295                 300

Val Arg Ala Ile Cys Leu Pro Glu Ala Asn Leu Gln Leu Pro Asp Trp
305                 310                 315                 320

Thr Glu Cys Glu Leu Ser Gly Tyr Gly Lys His Lys Ser Ser Ser Pro
                325                 330                 335

Phe Tyr Ser Glu Gln Leu Lys Glu Gly His Val Arg Leu Tyr Pro Ser
            340                 345                 350

Ser Arg Cys Ala Pro Lys Phe Leu Phe Asn Lys Thr Val Thr Asn Asn
        355                 360                 365

Met Leu Cys Ala Gly Asp Thr Arg Ser Gly Glu Ile Tyr Pro Asn Val
    370                 375                 380

His Asp Ala Cys Gln Gly Asp Ser Gly Gly Pro Leu Val Cys Met Asn
385                 390                 395                 400

Asp Asn His Met Thr Leu Leu Gly Ile Ile Ser Trp Gly Val Gly Cys
                405                 410                 415

Gly Glu Lys Asp Val Pro Gly Val Tyr Thr Lys Val Thr Asn Tyr Leu
            420                 425                 430

Gly Trp Ile Arg Asp Asn Met His Leu
        435                 440

## Description

Hemorrhagic stroke, or cerebral hemorrhage, is a form of stroke that occurs when a blood vessel in the brain ruptures or bleeds. Like ischemic strokes, hemorrhagic strokes interrupt the brain's blood supply because the bleeding vessel can no longer carry the blood to its target tissue. In addition, certain blood constituents irritate brain tissue, disrupting its delicately balanced regulation.By space occupation and the attendant increase in intracranial pressure, continuing bleeding will physically impinge on brain tissue and further restrict blood flow into the brain. In this respect, hemorrhagic strokes are more dangerous than their more common counterpart, ischemic strokes.

There are two types of hemorrhagic stroke: intracerebral hemorrhage, and subarachnoid hemorrhage. Intracerebral hemorrhage (ICH) denotes direct bleeding into the brain tissue, forming a gradually enlarging hematoma (pooling of blood) within the tissue. It generally occurs in small arteries or arterioles and is commonly due to hypertension, trauma, bleeding disorders, amyloid angiopathy, illicit drug use, and vascular malformations. The hematoma enlarges until its surround pressure limits its growth, or until it decompresses by emptying into the ventricular system, cerebrospinal fluid (CSF) or the pial surface. A third of intracerebral bleed is directed into the brain's ventricles (intraventricular hemorrhage; (IVH)). ICH has a mortality rate of 44 percent after 30 days, higher than ischemic stroke or even the highly dangerous subarachnoid hemorrhage.

Subarachnoid hemorrhage (SAH) is defined as bleeding into the cerebrospinal fluid of the subarachnoid space surrounding the brain. The two most common causes of SAH are rupture of aneurysms at the base of the brain and bleeding from vascular malformations near the pial surface. Bleeding into the CSF from a ruptured aneurysm occurs very quickly, causing a rapid lincrease of intracranial pressure. The bleeding usually only lasts a few seconds but rebleeding is common. Death or deep coma ensues if the bleeding continues. Hemorrhage from other sources is less abrupt and may continue for a longer period of time. SAH has a 40% mortality over a 30 day period.

Spontaneous intracerebral hemorrhage (ICH) is associated with significant morbidity and mortality. The mechanism of clinical deterioration are poorly understood, but may involve an increase of local tissue pressure as a result of mass effect, as well as the effects of toxic substances released from the hematoma, which may in turn reduce regional blood flow, compromise cell viability and finally cause cerebral ischemia, edema and cellular apoptosis. Part of the pathogenesis is a substantial release of glutamate and endogenous tissue plasminogen activator (t-PA), which, in turn, amplifies glutamate-induced damage. Reduced blood flow also facilitates the development of brain edema and, conversely, brain edema formation may restrict overall cerebral perfusion.

Both brain edema and decrease of cerebral blood flow are hallmarks of brain damage in ICH. Successful prevention of such damage depends upon a better understanding of the interactions between hematoma, intracranial hypertension, cerebral edema and reduction of cerebral blood flow, processes, which hence constitute central targets for future therapeutic avenues.

ICH accounts for about 10 - 15 % of all strokes in North America and Europe, and for about 20 - 30 % in East Asia. In general, ICH is classified according to the anatomic site of hemorrhage. Cortical (lobar) hematomas are distinguished from subcortical sites, such as basal ganglia, or brainstem. ICH may occur either with head injury or spontaneously.

Among spontaneous, non-traumatic hemorrhages, a distinction is made between primary and secondary ICH. Primary ICH in adults most commonly result from hypertensive arteriopathies, such as fibrinoid necrosis of lipohyalinosis in the brain. Since hypertensive arteriopathies develop predominantly in the basal perforating arteries, most hypertensive ICHs occur in the putamen, thalamus, pons and dentate nucleus of the cerebellum. In contrast, half of the primary cortical hemorrhages are non-hypertensive in nature and thought to arise from cerebral amyloid angiopathy or small vascular malformations. Other causative factors of secondary ICH include brain tumors, anticoagulants and thrombolytic agents. Also recreational drugs and sympathomimetics may precipitate secondary ICH.

Currently, from a clinical viewpoint, ICH is the most undesired type of stroke because mortality and morbidity are particularly high, and effective therapy is largely unavailable.

One approach for treating ICH is the surgical clot evacuation (craniotomy). However, in prospective randomized studies, craniotomy and subsequent clot removal provided no better clinical results than the best medical management possibly, because of the additional brain injury that may be produced during the transparenchymal approach to the clot.

More recently a minimal-invasive technique featuring stereotactic hematoma puncture, local fibrinolysis and subsequent aspiration of the liquefied clot has found interest. The lysis of clot remnants with e.g. rt-PA allows to reduce hematoma volume by 50 to 70 % with minimal associated brain damage. Promising clinical results in nonrandomized studies and experimental data showing positive effects on ischemia and the volume of early perihematomal edema support this therapeutic concept.

However, there have been reports of t-PA-mediated neurotoxic brain injury with delayed edema formation. Thus, data suggest that injection of t-PA for clot lysis promotes the development of delayed perihematomal edema, counteracting the beneficial effects of rapid hematoma volume decrease.

Thus, it is an objective of the present invention to provide novel means for treating hemorrhagic stroke, in particular intracerebral hemorrhage.

This objective is solved by providing a medicament for the patient suffering from hemorrhagic stroke comprising a therapeutically effective amount of a plasminogen activator (PA), which does not exert a neurotoxic effect within the patient. The neurotoxic effect is defined as an exaggerated activation of the NMDA type glutamate receptor. Thus, according to the invention, PAs are administered, which do not activate the NMDA type glutamate receptor within the patient.

In one embodiment of the invention the plasminogen activator is administered to the patient in combination with an effective amount of a neuroprotective. Such a neuroprotective may be used to prevent activation of the NMDA glutamate receptor by the plasminogen activator. Accordingly, plasminogen activators can be used, which have the potential to activate the glutamate receptor, and thus potentially are neurotoxic. However, due to the inhibitory effect of the neuroprotective contained within the medicament, the plasminogen activator in fact does not exert its neurotoxic potential within the patient.

Preferred examples for such neuroprotective substances are NMDA receptor antagonists, such as MK801 or, in a broader sense, agents able to antagonize activation of the NMDA receptor.

In a preferred embodiment of the invention, a non-neurotoxic plasminogen activator is used for treating a patient with hemorrhagic stroke, i.e. a plasminogen activator, which per se lacks the potential to activate the NMDA type glutamate receptor. This plasminogen activator favourably is essentially non-activatable by beta-amyloid or prion protein and in the presence of fibrin shows an enhanced activity of more than 550 fold, preferred more than 5500 fold, most preferred more than 10,000 fold, compared to the activity in the absence of fibrin. In a particular preferred embodiment the increase of activity of the PA in the presence of fibrin compared to its activity in the absence of fibrin is more than 100,000. Since the increase in activity of rt-PA is 550, the particularly preferred PA for the use according to the invention have an approximately 180-200 fold higher fibrin specificity/selectivity compared to rt-PA.

The preferred PA according to the invention have a half-life of more than 2.5 min, preferably more than 50 min, even more preferred more than 100 min.

The neurotoxicity can be assessed by methods known to the skilled person, e.g. with animal models in particular kainic acid models as described in detail in the international laid open WO03/037363. This model is further described in detail in Liberatore et al. (Liberatore, G.T.; Samson, A.; Bladin, C.; Schleuning, W.D.; Medcalf, R.L. "Vampire Bat Salivary Plasminogen Activator (Desmoteplase)", Stroke, February 2003, 537-543) and Reddrop et al. (Reddrop, C.; Moldrich, R.X.; Beart, P.M.; Liberatore, G.T.; Howells, D.W.; Schleuning, W.D.; Medcalf, R.L., "NMDA-mediated neurotoxicity is potentiated by intravenous tissue-type-, but not vampire bat-plasminogen activator, and is enhanced by fibrin", Monash University Department of Medicine, Version November 20, 2003).

In one preferred embodiment of the invention, DSPA alpha 1 or PA with a biological activity and pharmacological properties essentially corresponding to DSPA alpha 1 are used. DSPA alpha 1 has a half-life of 138 min and a 105,000 fold increased activity in the presence of fibrin compared to its activity in the absence of fibrin.

DSPA alpha 1 is a plasminogen activator, which originally was isolated or derived from the saliva of *Desmodus rotundus (**D**esmodus* **S**alivary **P**lasminogen **A**ctivator). Within the saliva four variants of DSPA had been isolated which, similarly to alteplase and urokinase, are composed of various conserved domains previously established in related families of proteins. The variants rDSPA alpha1 and rDSPA alpha2 exhibit the structural formula Finger (F), Epidermal Growth Factor (EGF) (E), Kringle (K), Protease (P), whereas rDSPA beta and rDSPA gamma are characterised by the formulas EKP and KP, respectively. Subtle sequence differences and data from southern blot hybridisation analysis indicate that the four enzymes are coded by four different genes and are not generated by differential splicing of a single primary transcript.

The variant DSPA alpha 1 has an at least 70% structural homology to alteplase (rt-PA); the difference being that alteplase has two kringles (FEKKP), whereas DSPA alpha 1 has only one (FEKP). DSPA alpha 1 is a serine protease with a molecular weight of 52 KD and 441 amino acids. Like other plasminogen activators (PA), DSPA alpha 1 activates plasminogen by catalysing the conversion of plasminogen into plasmin, which in turn breaks down the cross-linked fibrin abundant in blood clots.

DSPA alpha 1 has been found to have unique characteristics when compared to other thrombolytic agents, in particular with reference to alteplase. These include high specificity for plasminogen-bound fibrin, high fibrin selectivity (defined by activation by fibrin relative to activation by fibrinogen), no neurotoxicity, and negligible activation by beta-amyloid and human cellular prion protein, in addition to a long dominant half-life of more than 2 hours. All these characteristics result in a better safety and pharmacological profile for use in clinical settings not accessible for the thrombolytic agents currently available.

DSPA alpha 1 has been extensively studied *in vitro* and *in vivo,* using various thrombosis models and preferentially with alteplase as the comparator. In these studies, DSPA alpha 1 equalled or exceeded alteplase in potency, displaying shorter lysis times and lower reocclusion rates. Favourable pharmacokinetic properties appear to make an important contribution to its higher potency, in as much as DSPA alpha 1 has a lower total clearance and a longer terminal half-life than alteplase (see above).

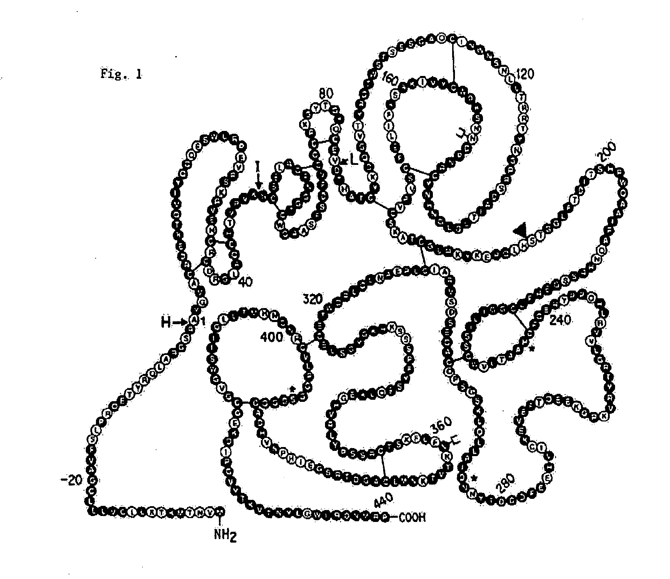

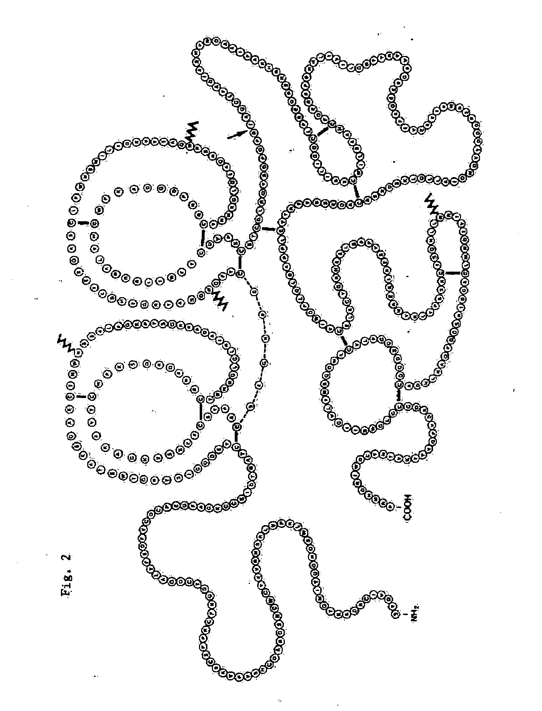

Recombinant DSPA alpha 1 is obtained from Chinese hamster ovary cells containing a recombinant plasmid carrying the DSPA alpha 1 gene from *Desmodus rotundus.* Fig. 1 and fig. 2 show the structures of DSPA alpha 1 and alteplase. The sequence of DSPA alpha 1 is shown in fig. 3.

The plasminogen activator from *Desmodus rotundus* and its recombinant form was first disclosed in the US patents US 6,008,019. The US 5,830,849 discloses the sequence data of DSPA alpha 1. Both patents are incorporated herein by reference in terms of the structure, properties and manufacture of plasminogen activators from *Desmodus rotundus,* in particular DSPA alpha 1 alpha 1.

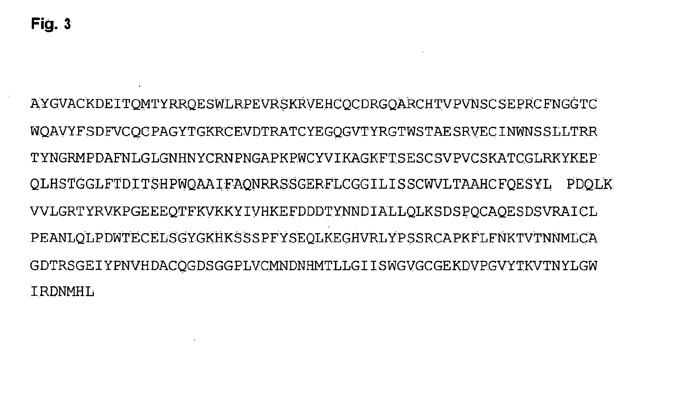

According to the present invention the term "desmoteplase" is used for any plasminogen activator with identical or essentially the same biological activity of DSPA alpha 1 regarding the activation of plasminogen and its enhanced fibrin selectivity/specificity. Preferably, the fibrin selectivity is at least 180 fold compared to rt-PA. The PA defined as desmoteplase according to the invention are preferably at least 80 or 90%, more preferred at least 95 %, most preferred at least 98% identical to the amino acid sequence according to fig. 3 (DSPA alpha 1). The plasminogen activators can include microheterogeneities, e.g. in terms if glycosylation and/or N-terminal variations, which are merely due to production systems.

According to the invention the term "hemorrhagic stroke" is defined as a bleeding within or into the brain or brain tissue, in particular intracerebral hemorrhage, intraventricular hemorrhage and subarachnoid hemorrhage.

The term "medicament" is defined as at least one pharmaceutical composition comprising at least one therapeutically effective plasminogen activator. In one embodiment the medicament may comprise an effective amount of a NMDA glutamate receptor inhibitor, which prevents the plasminogen activator from activating the NMDA glutamate receptor within the patient. The inhibitor can be comprised within the same preparation as the PA or can be contained in a separate preparation.

The neuroprotectant (i.e. NMDA glutamate receptor inhibitor) and the plasminogen activator can either be administered simultaneously as one single preparation or simultaneously or sequentially when contained in two separate preparations. The term sequentially within the scope of the invention refers to any method of administering the components which does not take place simultaneously. By simultaneous administration is meant the method of administration in which at least one component is administered for example by infusion over a longer period and the other component is also used during this period. If the two components are both administered by infusion over a longer period of time simultaneously means that the infusion periods overlap at least a short time.

According to the invention the plasminogen activators can be administered systemically by intravenous injection or locally. For the latter, in an advantageous embodiment of the invention, the PA are locally administered after a puncture of the hematoma, preferably guided by strereotaxic methodology. As for the finding of a human dose, the amount of PA to be applied can be determined by the size of the hemorrhage as determined by appropriate imaging methods, in a way that the volume of the hemorrhage defines the amount of the PA, e.g. DSPA alpha 1, to be administered. In a preferred embodiment the dosage of PA such as DSPA alpha 1 in mg equals to the hematoma size in cm³ as determined in T2^{*}.

### I. PORCINE MODEL OF ICH

To minimize the neurotoxic injury caused after intracerebral hemorrhage (ICH) by clot-derived substances in the surrounding brain tissue, minimally invasive neurosurgical protocols have evolved to evacuate the hematoma by stereotaxic injection of a fibrinolytic agent such as recombinant tissue plasminogen activator (rt-PA), followed by aspiration of the lysed clot.

A porcine model was developed to study the histopathological effects of recombinant tissue plasminogen activator on perihematomatous cell integrity. In pigs, lobar hematomas are induced by intracranial pressure (ICP)-controlled injections of a distinct amount of autologous blood into the white matter of the right frontal hemisphere. In the experimental group, the clots were lysed with rt-PA, thereby facilitating aspiration 20 min after hematoma induction. In control pigs, the hematoma resorption was allowed to follow its natural course. The rate of hematoma reduction and edema formation over 10 days was evaluated by means of planimetry of the MRI data and correlated to the histopathological changes found at autopsy.

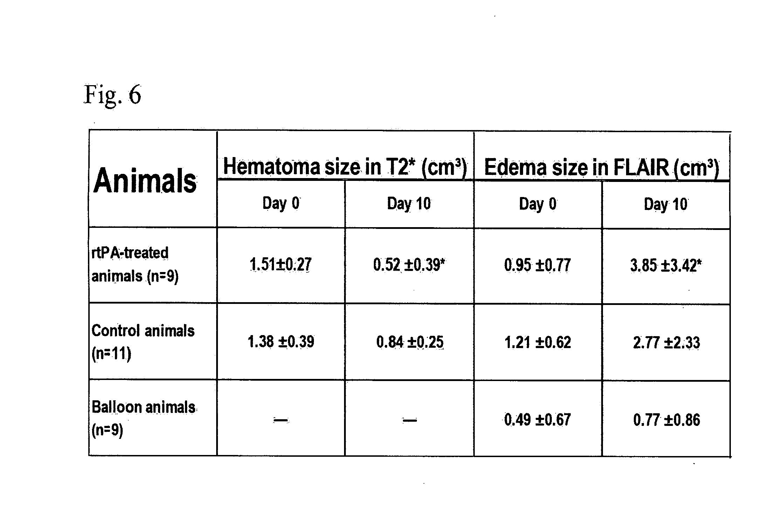

While rt-PA significantly accelerated clot resolution compared to controls (p<0.02), the increase of perihematomatous edema volume seen in controls on MRI within 3 and 10 days post-surgery was not ameliorated (Fig. 6). Controls and rt-PA-treated animals showed similar increases on day 3, while the increase from day 3 to day 10 was even twofold larger with rt-PA. Also earlier experiments were indicative of rt-PA-induced edema, showing a sustained increase in edema size over 10 days with rt-PA, but a biphasic time course in controls, with a shift towards normal on day 10 (Rohde, V.; Rohde, I.; Thiex, R; Ince, A.; Jung, A.; Duckers, G.; Groschel, K.; Rottger, C.; Kuker, W.; Müller, H.D.; Gilsbach, J.M.; "Fibrinolysis therapy achieved with tissue plasminogen activator and aspiration of the liquefied clot after experimental intracerebral hemorrhage: rapid reduction in hematoma volume but intensification of delayed edema formation", Journal of Neurosurgery, 2002, Volume 97, 954-962).

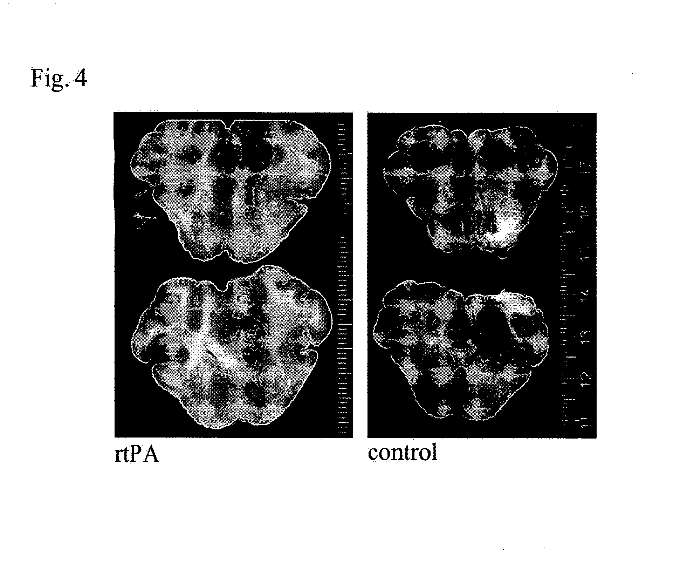

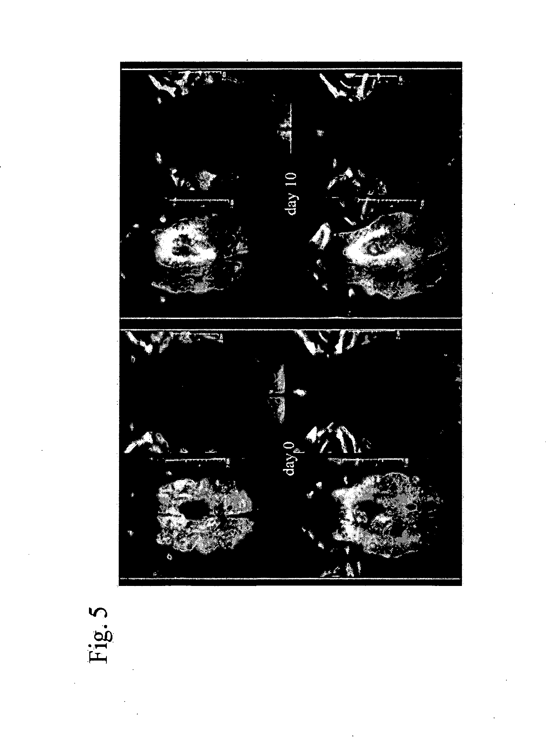

The gross pathology on day 10 and the edema formation on FLAIR 10 days after rt-PA therapy are shown in Fig. 4 and 5. The volumetry of MRI data are summarized in fig. 6. These data demonstrate the increase in delayed edema after fibrinolysis with rt-PA.

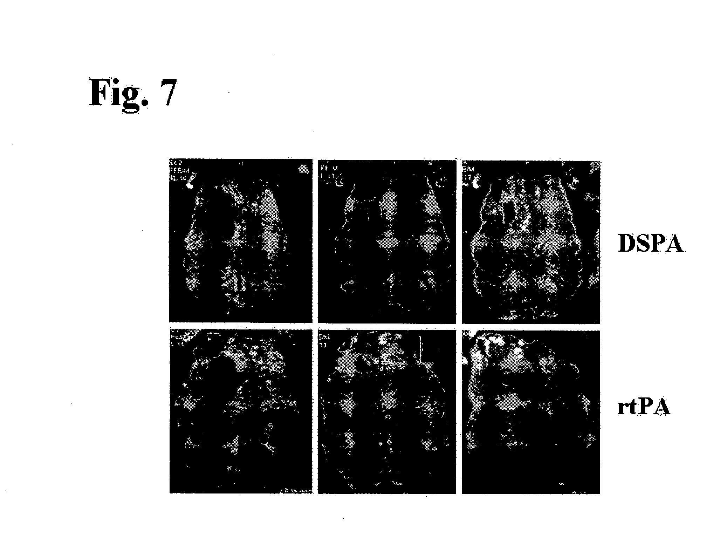

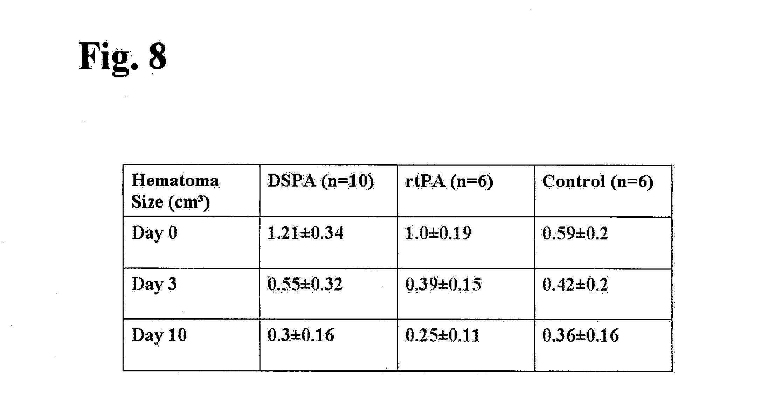

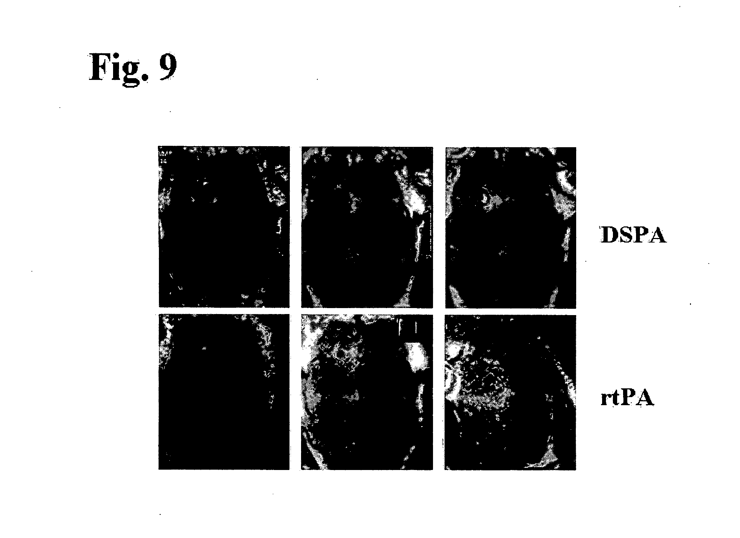

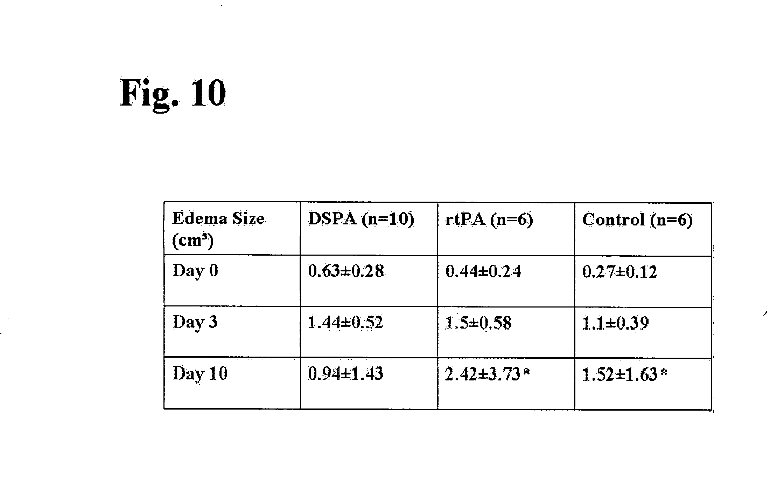

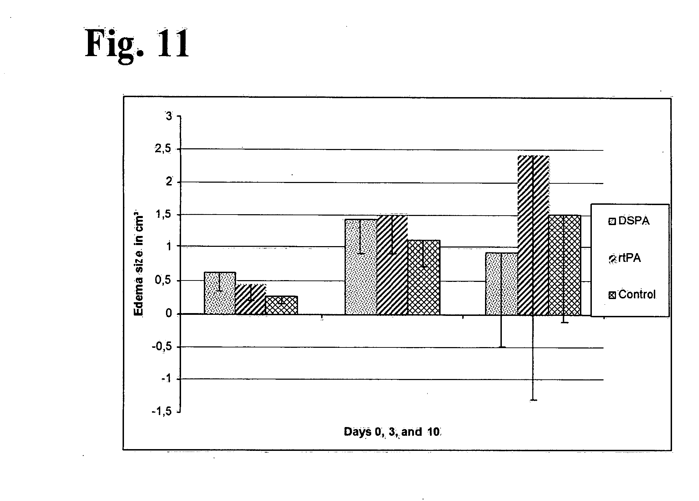

The hematoma size in T2* of DSPA alpha 1 or rt-PA treated animals is shown in fig. 7 and 8. The edema formation on FLAIR after both treatments is shown in fig. 9 and 10. The favorable development of edema formation after DSPA alpha 1 treatment is also shown in fig. 11. On day 3, controls and rt-PA or DSPA alpha 1 treatment groups showed similar increases in edema volume. This contrasts with the findings on day 10: The edema size decreased towards normal with DSPA alpha 1 (no significant difference between day 0 and day 10), whereas further increases were obtained in control and rt-PA-treated pigs. As a result, edema volumes in the latter two groups were significantly larger on day 10 than on day 0.

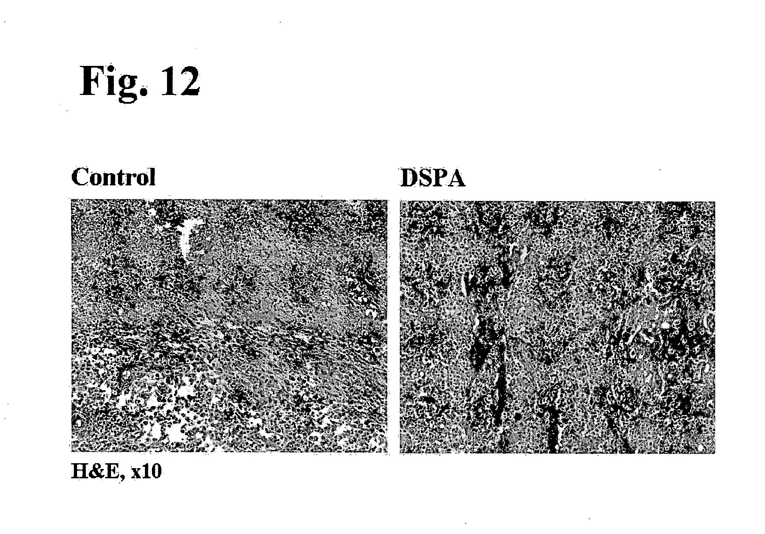

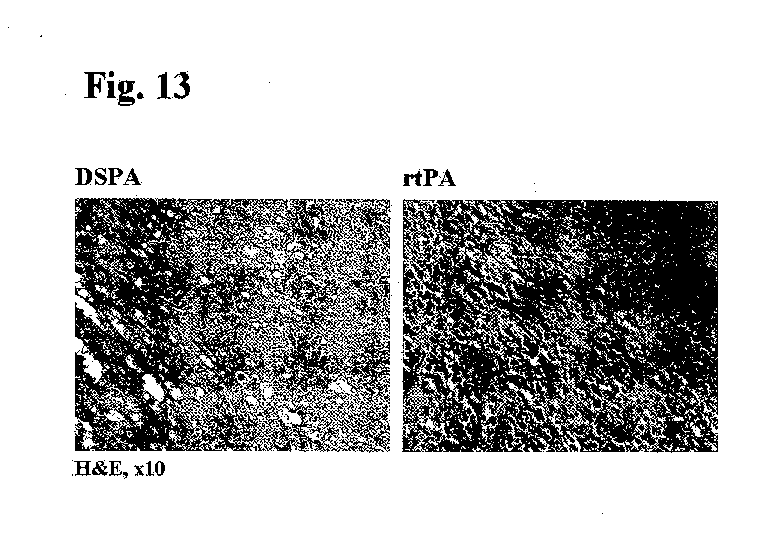

As a consistent finding, the extent of inflammatory infiltrates in histology was significantly less pronounced with DSPA alpha 1 than in animals treated with rt-PA. Fig. 12 shows the inflammatory reactions after spontaneous hematoma resorption vs. DSPA alpha 1 treatment. Fig. 13 shows the inflammatory reactions after local fibrinolysis using DSPA alpha 1 vs. rt-PA. In a semiquantitative score assessment of hematoxylin eosin-stained slices (3-tiered scale, '0' indicating mild, '1' focally accentuated, and '2' extensive inflammatory infiltrates reaching beyond the edema zone) DSPA-treated pigs reached a median of 0.5 compared with 1.5 in rtPA-treated pigs.

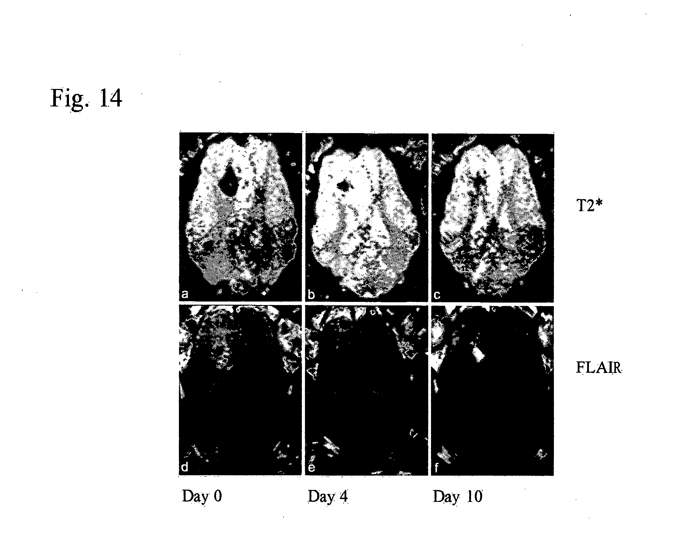

### Fig. 14, 15 and 16 show the advantageous effects of a combination of rt-PA and MK801:

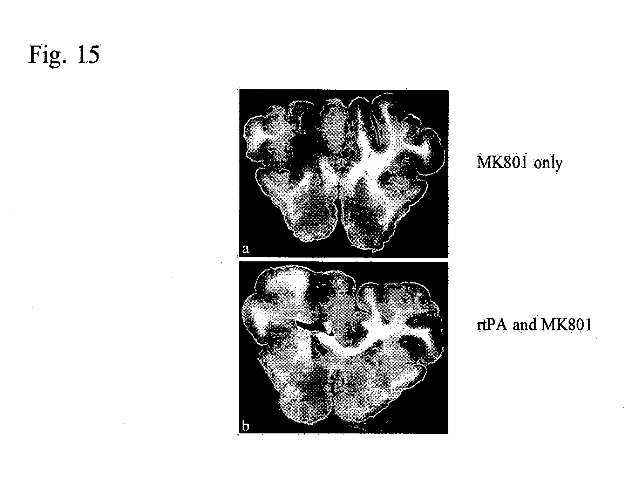

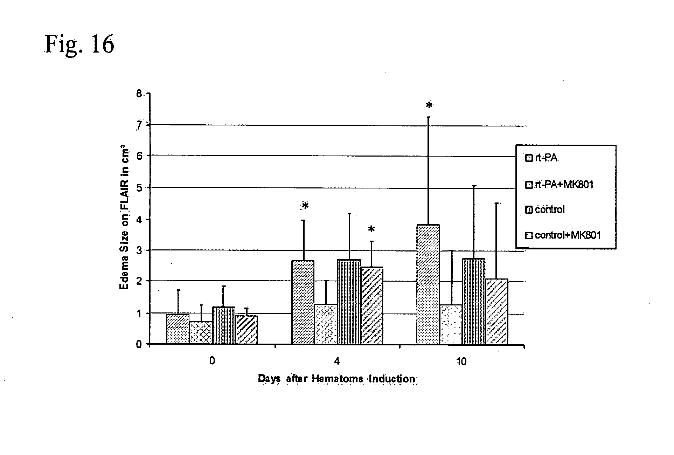

Fig. 14 demonstrates the reduction of the edema by MK-801; fig. 15 shows the hematoma reduction as reflected in gross pathology. The reduction of edema size on FLAIR in cm³ is quantified in fig. 16. These data demonstrate that MK801 blocks the promotion of delayed edema formation by rt-PA.

In conclusion, despite significant reduction in the size of the hematoma, clot liquefaction with rt-PA plus aspiration invokes a substantial edematous and inflammatory response within 10 days (for a detailed description of the method see Thiex, R.; Küker, W.; Müller, H. D.; Rohde, I.; Schröder, J. M.; Gilsbach, J. M.; Rohde, V., "The long-term effect of recombinant tissue-plasminogen-activator (rt-PA) on edema formation in a large-animal model of intracerebral hemorrhage", Neurological Research, 2003, Volume 25, April, pages 254-262).

Summarising these data the following conclusions can be drawn:
1. The extent of delayed edema formation after local fibrinolysis using DSPA alpha 1 is significantly less using DSPA alpha 1 compared with rt-PA.
2. On semiquantitative analysis of inflammatory infiltrates, DSPA alpha 1 treated pigs reach a median of 0.5 compared with 1.5 in rt-PA treated pigs.
3. As a benefit, it produces no delayed peri-hematomal edema (day 3 vs. day 10 and day 0 vs. day 10) and less inflammatory reactions.

## Claims

1. Use of a plasminogen activator for the manufacture of a medicament for the treatment of hemorrhagic stroke, wherein the plasminogen activator does not substantially activate the NMDA type glutamate receptor within the patient.

2. Use of a plasminogen activator according to claim 1, wherein the medicament comprises an inhibitor, which inhibits the NMDA type glutamate receptor within the patient.

3. Use of a plasminogen activator according to one of the above claims, wherein the inhibitor is an NMDA type glutamate receptor antagonist.

4. Use of a plasminogen activator according to one of the above claims, wherein the plasminogen activator has an at least more than 550 fold increased activity in the presence of fibrin compared to the activity without fibrin.

5. Use of a plasminogen activator according to one of the above claims, wherein the plasminogen activator
i. is essentially non-activatable by beta-amyloid and/or prion protein and/or
ii. is non-neurotoxic and/or
iii. has a half-life of at least more than 2.5 min.

6. Use of a plasminogen activator according to one of the above claims, wherein the plasminogen activator has an at least more than 5500 fold increased activity in the presence of fibrin compared to the activity without fibrin and has a half-life of at least more than 50 min.

7. Use of a plasminogen activator according to one of the above claims, wherein the plasminogen activator is desmoteplase.

8. Use of a plasminogen activator according to one of the above claims, wherein the plasminogen activator
i. has an amino acid sequence according to fig. 3 or microheterogeneous forms thereof or
ii. is at least 80 %, more preferred 95 %, even more preferred 98 % identical to the amino acid sequence of fig. 3.

9. Use of a plasminogen activator according to one of the above claims, wherein the hemorrhagic stroke is intracerebral hemorrhage, subarachnoid hemorrhage or intraventricular hemorrhage.

10. Use of a plasminogen activator according to one of the above claims, wherein the dosage of the plasminogen activator in mg equals to hematoma size in cm3 as determined in imaging systems, preferably MRI.
